# EUROPEAN PATENT SPECIFICATION

(11) **EP 1 844 797 B1**
(45) Date of publication and mention of the grant of the patent: **14.10.2009**
(21) Application number: 07007177.4
(22) Date of filing: 05.04.2007
(51) Int. Cl.: A61L 17/04, A61L 31/04, A61B 17/06

(54) **Yarns containing thermoplastic elastomer copolymer and polyolefin filaments**
Garne mit einem thermoplastischen Elastomer-Copolymer und Polyolefinfasern
Fils contenant un polymère en élastomère thermoplastique et des filaments de polyoléfine

(30) Priority: 06.04.2006 US 789950 P
(43) Date of publication of application: 17.10.2007
(73) Proprietor: Tyco Healthcare Group LP, Norwalk, Connecticut 06856 (US)
(72) Inventor: Hotter, Joseph, Middletown, CT 06457 (US); Cuevas, Brian J., Cumming, GA 30040 (US)
(74) Representative: HOFFMANN EITLE

(56) References cited:
- EP-A- 1 506 789
- EP-A1- 0 557 894
- GB-A- 2 038 704
- US-A1- 2005 125 036

## Description

### CROSS-REFERENCE TO RELATED APPLICATION

The present application claims the benefit of and priority to U.S. Provisional Patent Application Ser. No. 60/789,950 filed on April 6, 2006, the entire disclosure of which is incorporated by reference herein:

### BACKGROUND

### Technical Field

The present disclosure relates to yarns that contain at least one filament made from a thermoplastic elastomer copolymer and at least one filament made from a polyolefin material. Also disclosed are braided multifilaments suitably adapted for use as surgical devices and made from such yarns.

### Background of Related Art

Braided multifilaments often offer a combination of enhanced pliability, knot security and tensile strength when compared to their monofilament counterparts. The enhanced pliability of a braided multifilament is a direct consequence of the lower resistance to bending of a bundle of very fine filaments relative to one large diameter monofilament. However, a tradeoff between braid strength and pliability exists in the design of conventional braided multifilaments.

Braided multifilaments intended for the repair of body tissues should meet certain requirements: they should be substantially non-toxic, capable of being readily sterilized, they should have good tensile strength and pliability, they should also have acceptable knot-tying and knot-holding characteristics and if the braided multifilaments are of the bio-degradable variety, the degradation of the braided multifilaments should be predictable and closely controlled. When used in combination with a fixation device (e.g., bone screw or the like), the suture must be able to withstand the heat generated when the suture contacts the fixation device.

It would be advantageous to provide a braided multifilament suture that exhibits a combination of desirable characteristics.

### SUMMARY

The present disclosure describes yarns that contain at least one filament made from a thermoplastic elastomer copolymer such as, for example, a polyester-ether block copolymer and at least one filament made from a polyolefin material. The present disclosure also describes a heterogeneous yarn that includes a plurality of filaments made from a thermoplastic elastomer copolymer and at least one filament made from a polyolefin material. The heterogeneous yarns can be braided into a surgical article such as a suture or tape, or may be knitted or woven into a mesh.

The present disclosure describes a heterogeneous braid that includes a first yarn having at least one filament made from a thermoplastic elastomer copolymer and a second yarn having at least one filament made from a polyolefin material. The present disclosure also contemplates tapes, knits or weaves made from heterogeneous yarns including a thermoplastic elastomer copolymer and yarns made from a polyolefin material.

In certain embodiments, the heterogeneous braid or the braid made from one or more heterogeneous yarns are used to form surgical devices. In other embodiments, methods for approximating two tissue surfaces are contemplated. In one embodiment, a method of closing a wound in tissue includes the steps of passing said suture through the tissue and securing the ends of said suture to approximate the tissue, wherein the suture is made from first yarns and second yarns in a braided construction wherein the first yarns include a plurality of filaments comprising a thermoplastic elastomer copolymer , and the second yarns include a plurality of filaments comprising a polyolefin material. In another embodiment, a method of securing soft tissue to hard tissue includes the steps of: a. providing a surgical device fabricated from first yarns and second yarns in a braided construction wherein the first yarns include a plurality of filaments comprising a thermoplastic elastomer copolymer and the second yarns include a plurality of filaments comprising a polyolefin material; b. passing said surgical device through the soft tissue; c. securing said surgical device to the hard tissue; and d. manipulating said surgical device (e.g., by tying a knot in the device) to approximate the soft tissue and hard tissue. In yet another embodiment, a method of approximating hard tissues is contemplated, wherein a multifilament surgical device fabricated from a heterogeneous braid made from a first yarn and a second yarn in a braided construction wherein the first yarn includes a plurality of filaments comprising a thermoplastic elastomer copolymer and the second yarn includes a plurality of filaments comprising a polyolefin material is manipulated to approximate the hard tissues.

In yet other embodiments, the present disclosure relates to a surgical device that includes a suture anchor having at least one suture secured thereto, the suture having a braid including of a first set and a second set of continuous and discrete yarns in a braided construction. The first set of yarns are heterogeneous yarns containing first and second filaments wherein the first set of filaments are made from a thermoplastic elastomer copolymer and at least one of the second filaments is made from a polyolefin material.

### BRIEF DESCRIPTION OF THE DRAWINGS

The above and other objects, features, and advantages will become more readily apparent from the following description, reference being made to the accompanying drawings in which:
FIG. 1 is a schematic view of a heterogeneous yarn in accordance with one embodiment of this disclosure;
FIGS. 2A, 2B and 2C show illustrative embodiments of braids in accordance with one embodiment of this disclosure;
FIG. 3 shows a needle-suture combination that includes a suture made with a heterogeneous braid in accordance with one embodiment of this disclosure;
FIG. 4 is a perspective view of a suture, suture anchor and associated suture anchor driver as in one embodiment described herein;
FIG. 5 is an enlarged area of detail of Fig. 4;
FIG. 6 is a perspective view of a two part suture anchor being assembled with sutures of the present disclosure;
FIG. 7 is a perspective view of the suture anchor of FIG. 6 being positioned on an anchor driver;
FIG. 8 is a perspective view, partially shown in section, of the suture driver being rotated to drive the suture anchor carrying sutures in accordance with the present disclosure into bone; and
FIG. 9 is a cross-sectional view partially shown in perspective of the suture anchor and associated sutures installed through tissue and into bone.

### DETAILED DESCRIPTION

Filaments made from a thermoplastic elastomer copolymer and filaments made from a polyolefin material are used in accordance with the present disclosure to prepare yarns that can be incorporated into a braided, knitted, woven or other structure to provide a surgical device.

A plurality of filaments is used to form a yarn. A plurality of yarns is used to form a braid, knit or weave.

A "heterogeneous yarn" is a configuration containing at least two dissimilar filaments mechanically bundled together to form a yarn. The filaments are continuous and discrete, so therefore each filament extends substantially along the entire length of the yarn and maintains its individual integrity during yarn preparation, processing and use.

Unlike a heterogeneous yarn, a "homogeneous" yarn is a configuration containing substantially similar filaments. The filaments are also continuous and discrete. Therefore each filament extends substantially along the entire length of the yarn and maintains its individual integrity during yarn preparation, processing and use.

A "heterogeneous braid" is a configuration containing at least two dissimilar yarns. The two types of yarns are intertwined in a braided construction. The yarns are continuous and discrete, so therefore each yarn extends substantially along the entire length of the braid and maintains its individual integrity during braid preparation, processing and use.

In the broadest sense, this disclosure contemplates yarns that include at least one filament made from a thermoplastic elastomer copolymer and at least one filament made from a polyolefin material. This disclosure further contemplates the medical devices made from these yarns and their use in surgery.

It is envisioned that any thermoplastic elastomer copolymer known to one skilled in the art and capable of being spun into continuous filaments may be used. Particularly useful thermoplastic elastomer copolymer include polyester-ether block copolymers that contain at least a polyester hard segment and a polyether soft segment. These polyester-ether block copolymers can be formed of various configurations, i.e., AB, ABA, or BAB, wherein A is a polyester and B is a polyether.

Some examples of suitable polyesters include, but are not limited to polyalkylene terephthalate, polyethylene terephthalate and polybutylene terephthalate. Some examples of suitable polyethers include, but are not limited to long chain poly ether glycols, polyalkylene ether glycols, poly(ethylene ether)glycol, poly(1,2- and 1,3-propylene ether)glycol, poly(tetramethylene ether)glycol, poly(1,2-butylene ether)glycol, poly(pentamethylene ether)glycol, poly(hexamethylene ether)glycol, poly(heptamethylene ether)glycol, poly(octamethylene ether)glycol, and poly(nonamethylene ether)glycol. A particularly useful polyester-ether block copolymer contains polybutylene terephthalate and a long chain polyether glycol and is commercially available under the trademark HYTREL® from E. I. du Pont de Nemours and Company, Wilmington, Delaware.

It is also envisioned that any polyolefin known to one skilled in the art and capable of being spun into continuous filaments may be used. In useful embodiments the polyolefin material is made from polyethylene. In particularly useful embodiments, the polyethylene material is an ultra high molecular weight polyethylene. Ultra high molecular weight polyethylene ("UHMWP") is a linear polymer with an average molecular weight greater than about 400,000, typically in the range from about 500,000 to about 6,000,000. UHMWP has a high tenacity and low elongation rate to provide articles with greatly increased strength and decreased elongation.

Ultra high molecular weight polyethylene typically exhibits a very substantial degree of crystalline orientation (95-99%) and crystalline content (60-85%). The significant strength and stability of UHMWP is normally caused by the high degree of molecular orientation. As a result, the filaments exhibit strengths from about 375 kpsi (thousands of pounds per square inch) to about 560 kpsi, and tensile moduli of about 15 msi (millions of pounds per square inch) to about 30 msi. UHMWP is commercially available under the trademark SPECTRA® from Allied-Signal Technologies, Petersberg, Va. and under the trademark DYNEEMA® from DSM High Performance Fibers, JH Heerlen, The Netherlands.

The yarns may optionally contain filaments made of other materials. Materials used to construct these optional filaments can include a wide variety of natural and synthetic materials such as any materials previously known for the construction of sutures, meshes, sternal closure devices, cables, tapes and tethers. Such materials include bioabsorbable, non-bioabsorbable, biodegradable, non-biodegradable and bioerodible polymeric materials. Additionally carbon fibers, steel fibers, memory-shape alloys, silk, cotton, linen and other fibrous materials can also be employed.

Representative natural biodegradable polymers include polysaccharides such as alginate, dextran, cellulose, collagen, and chemical derivatives thereof (substitutions, additions of chemical groups, for example, alkyl, alkylene, hydroxylations, oxidations, and other modifications routinely made by those skilled in the art), and proteins such as albumin, zein and copolymers and blends thereof, alone or in combination with synthetic polymers.

Representative synthetic polymer blocks include polyphosphazenes, poly(vinyl alcohols), polyamides, polyester amides, poly(amino acid)s, synthetic poly(amino acids), polyanhydrides, polycarbonates, polyacrylates, polyalkylenes, polyacrylamides, polyalkylene glycols, polyalkylene oxides, polyvinyl halides, polyvinylpyrrolidone, polylactides, polyglycolides, polysiloxanes, polyurethanes and copolymers thereof.

Examples of suitable polyacrylates include poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate) and poly(octadecyl acrylate).

Synthetically modified natural polymers include cellulose derivatives such as alkyl celluloses, hydroxyalkyl celluloses, cellulose ethers, cellulose esters, nitrocelluloses, and chitosan. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate and cellulose sulfate sodium salt. These are collectively referred to herein as "celluloses".

Representative synthetic degradable polymers include polyhydroxy acids, such as polylactides, polyglycolides and copolymers thereof; poly(hydroxybutyric acid); poly(hydroxyvaleric acid); poly(lactide-co-(ε-caprolactone-)); poly(glycolide-co-(ε-caprolactone)); polycarbonates, poly(pseudo amino acids); poly(amino acids); poly(hydroxyalkanoate)s; polyanhydrides; polyortho esters; and blends and copolymers thereof.

Examples of non-biodegradable polymers include ethylene vinyl acetate, poly(meth)acrylic acid, polyamides, polystyrene, polyvinyl chloride, polyvinylphenol, and copolymers and mixtures thereof.

Rapidly bioerodible polymers such as poly(lactide-co-glycolide)s, polyanhydrides, and polyorthoesters, which have carboxylic groups exposed on the external surface as the smooth surface of the polymer erodes, also can be used.

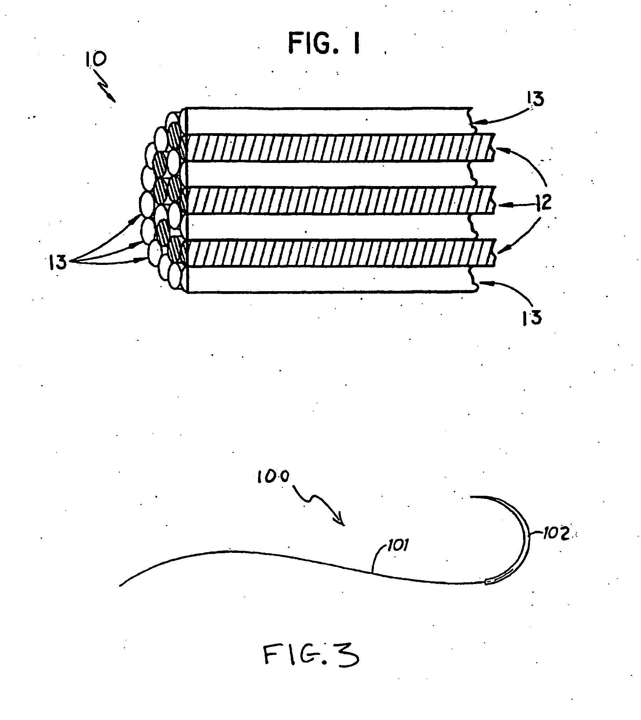

In one embodiment, a heterogeneous yarn 10 contains a plurality of two dissimilar filaments as shown in Figure 1. First filaments 12 are made from a thermoplastic elastomer copolymer and second filaments 13 are made from a polyolefin material. A plurality of the two dissimilar filaments are commingled to form a heterogeneous yarn.

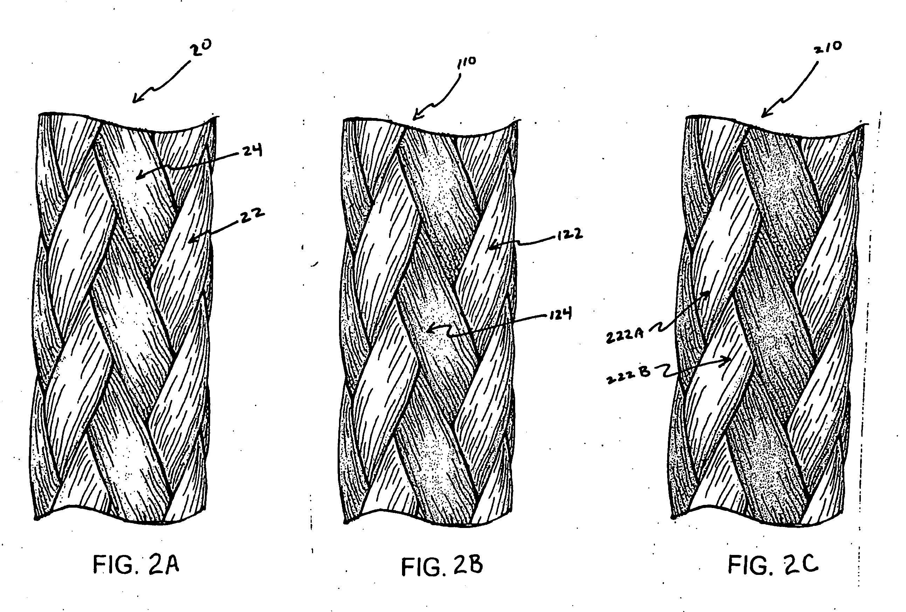

In another embodiment shown in Figure 2A, a heterogeneous braid 20 contains two dissimilar yarns. A first yarn 22 contains a plurality of filaments made from a thermoplastic elastomer copolymer. A second yarn 24 contains a plurality of filaments made from a polyolefin material. The first and second yarns are intertwined to form a heterogeneous braid.

In still another embodiment shown in Figure 2B, a heterogeneous braid 120 contains a heterogeneous yarn 122 and a homogeneous yarn 124. As described above, a heterogeneous yarn contains a plurality of two dissimilar filaments. Preferably, a first filament is made from a thermoplastic elastomer copolymer and a second filament is made from a polyolefin material. A homogeneous yarn contains a plurality of filaments made from any material capable of being spun into a filament. The heterogeneous yarn and the homogeneous yarn are intertwined to form a heterogeneous braid.

In yet another embodiment shown in Figure 2C, a braid 210 contains two similar heterogeneous yarns 222A, 222B. Each heterogeneous yarn contains a plurality of two dissimilar filaments. Preferably, a first filament is made from a thermoplastic elastomer copolymer and a second filament is made from a polyolefin material. The heterogeneous yarns are intertwined to form a braid.

Particularly useful filament materials include a polyester-ether block copolymer, such as HYTREL®, and a polyolefin, such as an ultra high molecular weight polyethylene like SPECTRA®.

A heterogeneous braid and/or yarn can be prepared using conventional braiding technology and equipment commonly used in the textile industry, and in the medical industry for preparing multifilament sutures. Suitable braid constructions are disclosed, for example, in U.S. Pat. Nos. 3,187,752; 3,565,077; 4,014,973; 4,043,344; 4,047,533; 5,019,093; and 5,059,213, the disclosures of which are incorporated herein by reference. Illustrative flat braided structures (suitable, e.g., for tendon repair) which can be formed using the presently described heterogeneous yarns include those described in U.S. Patent Nos. 4,792,336 and 5,318,575. Suitable mesh structures are shown and described, for example, in Hain et al. U.S. Patent No. 5,292,328. In addition, shape memory fibers may be incorporated into non-woven structures, such as felt. One suitable non-woven structure is shown and described in Koyfman et al. U.S. Patent No. 5,393,534.

If desired, the surface of a filament, yarn or braid can be coated with a bioabsorbable or nonabsorbable coating to further improve the performance of the braid. For example, a braid can be immersed in a solution of a desired coating polymer in an organic solvent, and then dried to remove the solvent.

If the surface of a filament, yarn or braid is coated, then the coating composition may desirably contain bioactive materials. Some examples include: vasoactive agents, neuroactive agents, hormones, growth factors, cytokines, anaesthetics, steroids, anticoagulants, anti-inflammatories, immunomodulating agents, cytotoxic agents, prophylactic agents, antibiotics, antimicrobial, antivirals, antisense, antigens and antibodies.

A heterogeneous braid is sterilized so it can be used for a host of medical applications, especially for use as a surgical suture, cable, tether, tape and sternal closure device, preferably attached to a needle, suture anchor, or bone anchor. For example, as shown in Figure 3, a needle-suture combination 100 includes a suture 101 made from a heterogeneous yarn in accordance with this disclosure attached to a needle 102. A braid can be sterilized using any of the conventional techniques well known in the art.

Once sterilized, a braided multifilament surgical device, as described herein, may be used to repair wounds located between two or more soft tissues, two or more hard tissues, or at least one soft tissue and at least one hard tissue. The braided multifilament surgical device is passed through, wrapped around or secured to tissue and then the tissue is approximated by manipulating the braided multifilament surgical device, such as, for example, by tying a knot, cinching the device, applying a buckle, or the like.

In embodiments, a braid is made of heterogeneous yarns to form a surgical suture. Preferably, the heterogeneous yarns contain filaments made from a thermoplastic elastomer copolymer and filaments made from a polyolefin material. The thermoplastic elastomer copolymer filaments preferably comprise from about 10% to about 90% of the cross-sectional area of the heterogeneous yarns, more preferably from about 25% to 75%, and most preferably from about 25% to 50% of the heterogeneous yarns.

The polyolefin material filaments preferably comprise from about 10% to about 90% of the cross-sectional area of the heterogeneous yarns, more preferably from about 25% to 75%, and most preferably from about 25% to 50% of the heterogeneous yarns.

Sutures made in accordance with the foregoing description will exhibit superior strength and resistance to abrasion, and may find particular use in cardiac surgery and orthopedic surgery. With respect to orthopedic surgery in particular, the suture will be useful in securing bone under high stress and abrasion. The present multifilament braided sutures can advantageously used in combination with a fixation device, including, but not limited to, for example, suture anchors.

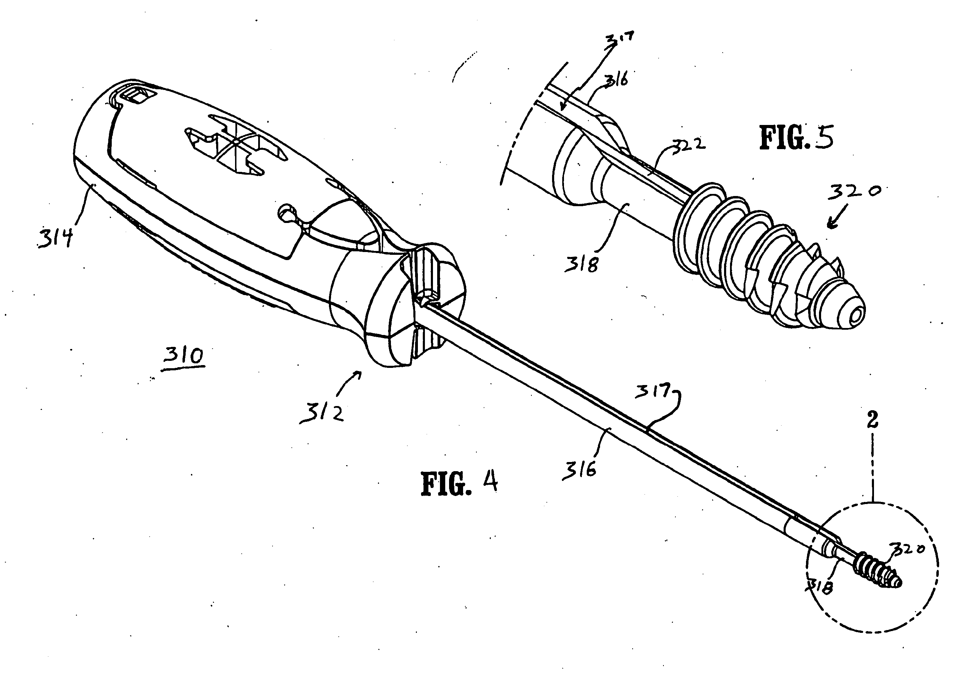

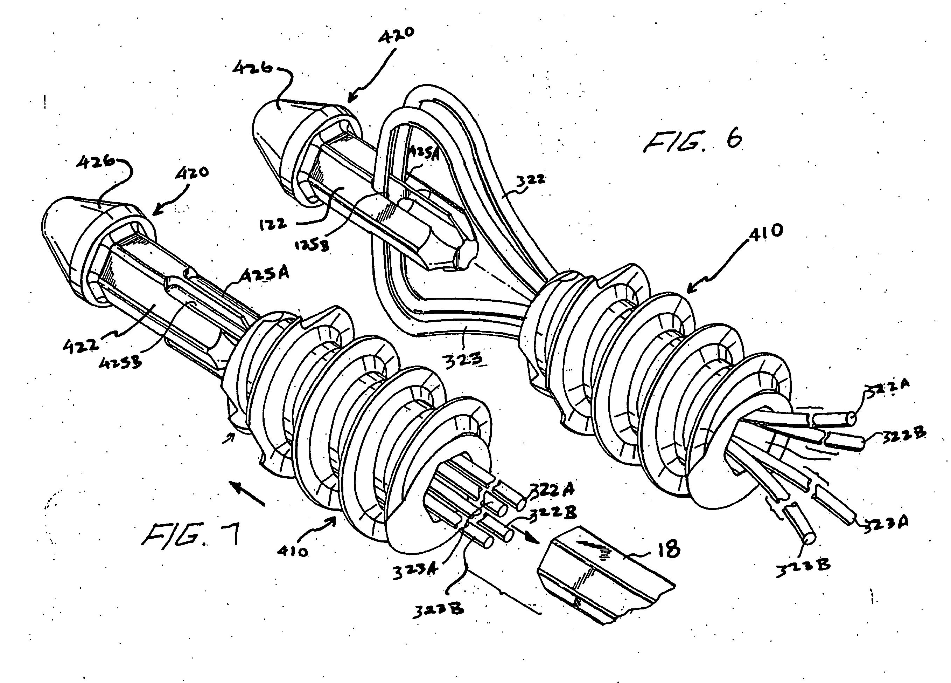

In a particularly useful embodiment, it is contemplated that the suture in accordance with the disclosure may be delivered in conjunction with a suture anchor delivery system and may be passed through tissue using an arthroscopic suturing instrument. Referring now to Figs. 4 and 5, one suitable suture anchor delivery system 310 is shown having a handle 314 with an elongate shaft 316 supporting a threaded suture anchor 320 at the distal tip 318 of the shaft 316 away from the handle 314. As shown in Fig. 5, suture 322 made in accordance with the present disclosure is attached to the suture anchor 320 and is led through trough 317 in the shaft 316 (and a corresponding trough (not shown) on the other side shaft 316) to handle 314. Referring now to Figs. 6 and 7, one method of pre-attaching a pair of sutures 322, 323 to a suture anchor is shown. As shown, suture anchor 320 consists of two parts, a hollow-threaded body portion 410 and a tip portion 420 having a shaft 422 insertable into the hollow body portion 410 and configured to receive and hold two sutures 322, 323 through transverse apertures 425A, 425B, as shown. Enlarged tip bead section 426 does not pass into or through the hollow threaded body 410, thereby retaining the suture relative to the suture anchor as the sutures 322, 323 are placed under tension by pulling on proximal ends 322A, 322B, 323A, 323B. Of course, numerous other types of suture anchors and methods of attaching sutures and in accordance with the present disclosure will occur to those skilled in the art. By way of example, the suture alternatively may be attached to a push-in-type anchor rather than a screw-in type anchor. See for example, Larsen U.S. Patent No. 5,993,459. Preferably, the proximal ends of the suture are attached to needles (not shown) suitable for use during surgery to pass the suture through tissue and, via appropriate manipulation (e.g., knot tying and/or cinching) secure the tissue relative to the anchor.

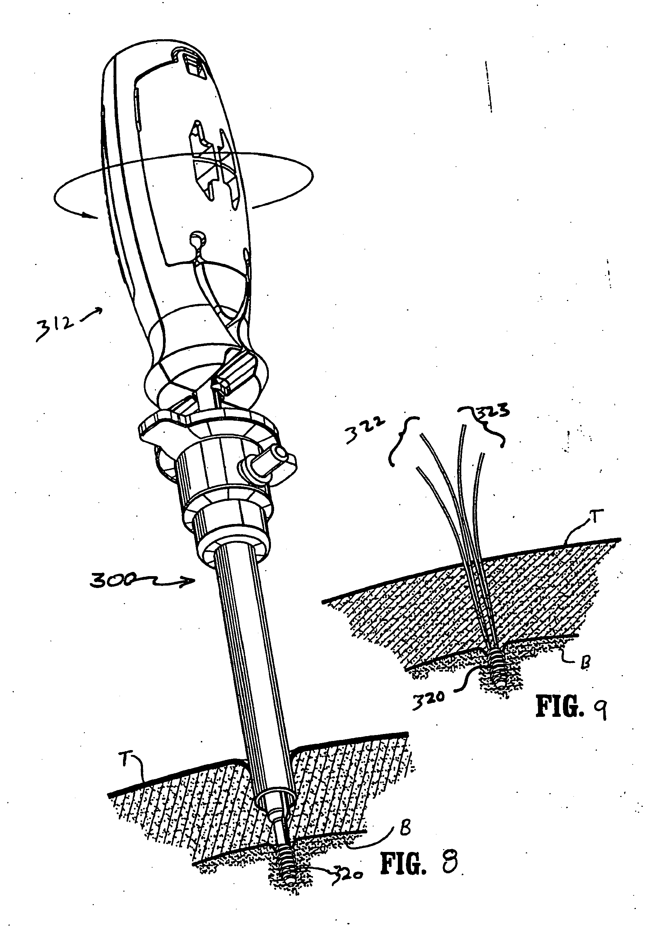

In use during an arthroscopic procedure a cannula 300 is inserted into the joint capsule and the shaft 316 of the suture anchor delivery system is inserted through the cannula 300 to a prepared site suitable to receive suture anchor. Fig. 8 shows the shaft of the instrument inserted through cannula 430 with the suture anchor 320 inserted into bone B. The suture anchor 320 is released from the delivery system 310 leaving the sutures 322, 323 available for manipulation to secure the soft tissue T to bone B. In a further embodiments the sutures are attached to needles (not shown) suitable for passing through soft tissue. The needles may be traditional suture needles suitable for use with an arthroscopic suturing instrument. One such instrument is the Arthrosew instrument (U.S.S. Sports Medicine, North Haven, CT) which utilizes a double ended surgical incision mender. Most preferably, the handle portion of the suture anchor delivery system includes releasable suture management members (not shown) which hold the suture needles for use. If the suture needles are to be used with a suturing device, the suture management members are configured to engage the suturing instrument in a suitable manner to transfer control of the needle to the suturing instrument. The needles and suture(s) are passed through soft tissue and the suture is manipulated, such as by forming in the suture, to secure the soft tissue relative to the suture anchor. Thereafter, the patient is closed in a suitable manner depending upon whether the procedure was conducted as an open, mini-open or closed arthroscopic approach.

In the context of a suture anchor, a suture constructed in accordance with the present disclosure provides significantly enhanced resistance to abrasion as the suture is manipulated, including drawing the suture through the suture eyelets of the suture anchor, forming knots in the suture, and cinching the knots down tightly for secure approximation of the soft tissue to bone.

Various modifications and variations of the yarns, braids and devices and uses thereof will be apparent to those skilled in the art from the foregoing detailed description.

## Claims

1. A surgical device comprising first yarns and second yarns in a braided construction, wherein the first yarns comprise at least one filament comprising a thermoplastic elastomer copolymer, and the second yarns comprise at least one filament comprising a polyolefin material.

2. The surgical device according to claim 1, wherein the thermoplastic elastomer copolymer comprises a polyester-ether block copolymer including a polybutylene terephthalate and a long chain polyether glycol, and the polyolefin material comprise polyethylene.

3. The surgical device according to claim 1 or 2, wherein the first yarns and the second yarns are multifilament yarns.

4. The surgical device according to any one of the preceding claims, wherein all the filaments of the first yarn comprise a thermoplastic elastomer copolymer and all the filaments of the second yarn comprise a polyolefin material.

5. A heterogeneous yarn comprising at least one filament comprising a thermoplastic elastomer copolymer, and at least one filament comprising a polyolefin material.

6. A heterogeneous yarn according to claim 5, wherein the thermoplastic elastomer copolymer comprises a polyester-ether block copolymer including a polybutylene terephthalate and a long chain polyether glycol, and the polyolefin material comprises polyethylene.

7. A surgical device comprising a heterogeneous yarn in accordance with claim 5 or 6.

8. A sterile braid comprising a heterogeneous yarn in accordance with claim 5 or 6.

9. A multifilament surgical device comprising:
a plurality of heterogeneous yarns, each heterogeneous yarn including first and second filaments; and
the plurality of heterogeneous yarns being in a braided construction,
wherein the first filaments comprise a thermoplastic elastomer copolymer, and the second filaments comprise a polyolefin material.

10. The multifilament surgical device according to claim 9, wherein the thermoplastic elastomer copolymer comprises a polyester-ether block copolymer including a polybutylene terephthalate and a long chain polyether glycol, and the polyolefin material comprises polyethylene.

11. A multifilament surgical device comprising a braid comprising:
a first set and a second set of continuous and discrete yarns in a braided construction; and
the first set of yarns comprising heterogeneous yarns including first and second filaments,
wherein the first set of filaments comprise a thermoplastic elastomer copolymer, and at least one of the second filaments comprises a polyolefin material.

12. The multifilament surgical device according to claim 11, wherein the second set of yarns comprise heterogeneous yarns including first and second filaments wherein the first set of filaments comprise a thermoplastic elastomer copolymer, and at least one of the second filaments comprises a polyolefin material.

13. The multifilament surgical device according to claim 11 or 12, wherein the second set of yarns comprise homogeneous yarns and the surgical device optionally includes a bioabsorbable, non-bioabsorbable, biodegradable, non-biodegradable, or bioerodible polymeric material.

14. The multifilament surgical deice according to any one of claims 9 to 13, wherein the surgical device comprises a suture secured to a suture anchor.

15. The multifilament surgical device according to any one of claims 9 to 14, wherein the thermoplastic elastomer copolymer comprises a polyester-ether block copolymer including a polybutylene terephthalate and a long chain polyether glycol, and the polyolefin material comprises polyethylene.

## Patentansprüche

1. Chirurgische Vorrichtung, umfassend erste Garne und zweite Garne in einer geflochtenen Konstruktion, wobei die ersten Garne zumindest ein Filament mit einem thermoplastischen Elastomer-Copolymer aufweisen und die zweiten Garne zumindest ein Filament mit einem Polyolefinmaterial aufweisen.

2. Chirurgische Vorrichtung nach Anspruch 1, wobei das thermoplastische Elastomer-Copolymer ein Polyester-Äther Block-Copolymer mit einem Polybutylenterephthalat und einem langkettigen Polyesterglycol aufweist und das Polyolefinmaterial Polyethylen aufweist.

3. Chirurgische Vorrichtung nach Anspruch 1 oder 2, wobei die ersten Garne und die zweiten Garne Multifilamentgarne sind.

4. Chirurgische Vorrichtung nach einem der vorstehenden Ansprüche, wobei alle Filamente des ersten Garns ein thermoplastisches Elastomer-Copolymer und alle Filamente des zweiten Garns ein Polyolefinmaterial aufweisen.

5. Heterogenes Garn, umfassend zumindest ein Filament mit einem thermoplastischen Elastomer-Copolymer und zumindest ein Filament mit einem Polyolefinmaterial.

6. Heterogenes Garn nach Anspruch 5, wobei das thermoplastische Elastomer-Copolymer ein Polyester-Äther Block-Copolymer mit einem Polybutylenterephthalat und ein langkettiges Polyesterglycol enthält und das Polyolefinmaterial Polyethylen enthält.

7. Chirurgische Vorrichtung, umfassend ein heterogenes Garn gemäß Anspruch 5 oder 6.

8. Sterile Schnur, umfassend ein heterogenes Garn gemäß Anspruch 5 oder 6.

9. Chirurgische Multifilamentvorrichtung, umfassend:
eine Mehrzahl von heterogenen Garnen, wobei jedes heterogene Garn erste und zweite Filamente enthält; und
wobei die Mehrzahl von heterogenen Garnen in einer geflochtenen Konstruktion vorliegt,
wobei die ersten Filamente ein thermoplastisches Elastomer-Copolymer und die zweiten Filamente ein Polyolefinmaterial aufweisen.

10. Chirurgische Multifilamentvorrichtung nach Anspruch 9, wobei das thermoplastische Elastomer-Copolymer ein Polyester-Äther Block-Copolymer mit einem Polybutylenterephthalat und einem langkettigen Polyesterglycol enthält, und das Polyolefinmaterial Polyethylen aufweist.

11. Chirurgische Multifilamentvorrichtung, umfassend eine Schnur, die umfasst:
einen ersten Satz und einen zweiten Satz von kontinuierlichen und diskreten Garnen in einer geflochtenen Konstruktion; und
wobei der erste Satz von Garnen heterogene Garne mit ersten und zweiten Filamenten enthält,
wobei der erste Satz von Filamenten ein thermoplastisches Elastomer-Copolymer aufweist und zumindest eines der zweiten Filamente ein Polyolefinmaterial aufweist.

12. Chirurgische Multifilamentvorrichtung nach Anspruch 11, wobei der zweite Satz von Garnen heterogene Garne mit ersten und zweiten Filamenten aufweist, wobei der erste Satz von Filamenten ein thermoplastisches Elastomer-Copolymer aufweist und zumindest eines der zweiten Filamente ein Polyolefinmaterial aufweist.

13. Chirurgische Multifilamentvorrichtung nach Anspruch 11 oder 12, wobei der zweite Satz von Garnen homogene Garne aufweist und die chirurgische Vorrichtung optional ein bioabsorbierbares, nicht bioabsorbierbares, biologisch abbaubares, nicht biologisch abbaubares oder biologisch auswaschbares Polymermaterial enthält.

14. Chirurgische Multifilamentvorrichtung nach einem der Ansprüche 9 bis 13, wobei die chirurgische Vorrichtung einen Nähfaden aufweist, der an einem Fadenanker befestigt ist.

15. Chirurgische Multifilamentvorrichtung nach einem der Ansprüche 9 bis 14, wobei das thermoplastische Elastomer-Copolymer ein Polyester-Äther Block-Copolymer mit einem Polybutylenterephthalat und einem langkettigen Polyesterglycol enthält, und das Polyolefinmaterial Polyethylen aufweist.

## Revendications

1. Dispositif chirurgical comprenant des premiers fils et des deuxième fils dans une construction tressée, où les premiers fils comprennent au moins un filament comprenant un copolymère d'élastomère thermoplastique, et les deuxièmes fils comprennent au moins un filament comprenant un matériau de polyoléfine.

2. Dispositif chirurgical selon la revendication 1, dans lequel le copolymère d'élastomère thermoplastique comprend un copolymère bloc polyester-éther incluant un polybutylène térephthalate et un glycol de polyéther à chaîne longue, et le matériau de polyoléfine comprend le polyéthylène.

3. Dispositif chirurgical selon la revendication 1 ou 2, dans lequel les premier fils et les deuxième fils sont des fils multifilaments.

4. Dispositif chirurgical selon l'une quelconque des revendications précédentes, dans lequel tous les filaments du premier fil comprennent un copolymère d'élastomère thermoplastique et tous les filaments du deuxième fil comprennent un matériau de polyoléfine.

5. Fil hétérogène comprenant au moins un filament comprenant un copolymère d'élastomère thermoplastique, et au moins un filament comprenant un matériau de polyoléfine.

6. Fil hétérogène selon la revendication 5, dans lequel le copolymère d'élastomère thermoplastique comprend un copolymère bloc polyester-éther incluant un polybutylène téréphtalate et un glycol de polyéther à chaîne longue, et le matériau de polyoléfine comprend le polyéthylène.

7. Dispositif chirurgical comprenant un fil hétérogène selon la revendication 5 ou 6.

8. Tresse stérile comprenant un fil hétérogène selon la revendication 5 ou 6.

9. Dispositif chirurgical multifilaments comprenant:
une pluralité de fils hétérogènes, chaque fil hétérogène incluant des premier et deuxième filaments; et
la pluralité de fils hétérogènes étant dans une construction tressée,
où les premier filaments comprennent un copolymère d'élastomère thermoplastique, et les deuxième filaments comprennent un matériau de polyoléfine.

10. Dispositif chirurgical multifilament selon la revendication 9, dans lequel le copolymère d'élastomère thermoplastique comprend un copolymère bloc de polyester-éther incluant un polybutylène téréphtalate et un glycol de polyéther à chaîne longue, et le matériau de polyoléfine comprend le polyéthylène.

11. Dispositif chirurgical multifilament comprenant une tresse comprenant:
un premier ensemble et un deuxième ensemble de fils continus et discrets dans une construction tressée; et
le premier ensemble de fils comprenant des fils hétérogènes incluant des premier et deuxième filaments,
où le premier ensemble de filaments comprennent un copolymère d'élastomère thermoplastique, et au moins un des deuxième filaments comprend un matériau de polyoléfine.

12. Dispositif chirurgical multifilament selon la revendication 11, dans lequel le deuxième ensemble de fils comprennent des fils hétérogènes incluant des premier et deuxième filaments, le premier ensemble de filaments comprenant un copolymère d'élastomère thermoplastique, et au moins un des deuxième filaments comprend un matériau de polyoléfine.

13. Dispositif chirurgical multifilament selon la revendication 11 ou 12, dans lequel le deuxième ensemble de fils comprennent des fils homogènes, et le dispositif chirurgical comprend en option un matériau polymère bioabsorbable, non-bioabsorbable, biodégradable, non biodégradable ou bioérodible.

14. Dispositif chirurgical multifilament selon l'une quelconque des revendications 9 à 13, dans lequel le dispositif chirurgical comprend un fil de suture fixé à un élément d'ancrage de fil de suture.

15. Dispositif chirurgical multifilament selon l'une quelconque des revendications 9 à 14, dans lequel le copolymère d'élastomère thermoplastique comprend un copolymère bloc polyester-éther incluant un polybutylène téréphtalate et un glycol de polyéther à chaîne longue, et le matériau de polyoléfine comprend le polyéthylène.
